# EUROPÄISCHE PATENTSCHRIFT

(11) **EP 1 621 132 B1**
(45) Veröffentlichungstag und Bekanntmachung des Hinweises auf die Patenterteilung: **07.03.2007**
(21) Anmeldenummer: 04002436.6
(22) Anmeldetag: 04.02.2004
(51) Int. Cl.: A61B 5/15

(54) **Vorrichtung und Verfahren zur Probenahme und Analyse vor Körperflüssigkeiten**
Device and method for sampling and analysing body fluids
Dispositif et procédé pour le prélèvement et l'analyse de liquides corporels

(30) Priorität: 27.06.2003 EP 03014772
(43) Veröffentlichungstag der Anmeldung: 01.02.2006
(73) Patentinhaber: Ehrfeld Mikrotechnik AG in Insolvenz, 55234 Wendelsheim (DE)
(72) Erfinder: Ehrfeld, Wolfgang, Prof. Dr., 55124 Mainz (DE); Linnebach, Egbert, Dr., 64572 Büttelborn (DE); Nagy, Karoly, Prof., 52070 Aachen (DE); Azzawi, Alexander, Dr., 55129 Mainz (DE); Kammermeier, Stefan, Dr., 55234 Erbes-Büdesheim (DE); Schwank, Thomas, 56179 Vallendar (DE)
(74) Vertreter: Meyer-Dulheuer, Karl-Hermann

(56) Entgegenhaltungen:
- WO-A-02/100251
- WO-A-02/100252
- DE-A- 3 708 031
- US-A- 4 637 403
- US-A- 5 505 212
- US-A1- 2002 198 444
- US-A1- 2003 088 191

## Beschreibung

Gegenstand der Erfindung ist eine Vorrichtung mit denen die Probenahme einer Körperflüssigkeit, beispielsweise Blut, sowie die quantitative Analyse darin enthaltener Bestandteile durchgeführt werden kann.

In der klinischen Diagnostik ist die Untersuchung von Körperflüssigkeiten, insbesondere von Blut, eine wichtige Methode, um den Gesundheitszustand eines Patienten zu überprüfen. Die häufigsten Untersuchungen werden dabei im Bereich Homecare mit Kapillarblut vom Patienten selbst durchgeführt. Für solche Anwendungen, vor allem für die Bestimmung des Glucosespiegels im Blut, verwenden die Patienten Einstechhilfen, um die Haut leicht zu verletzen und einen kleinen Blutstropfen zu erhalten. Diese Blutprobe wird dann in der Regel auf einen Teststreifen aufgetragen, der mit Hilfe eines Messgeräts ausgewertet wird. Um diese umständliche Prozedur zu vereinfachen und den Schmerz des Patienten zu minimieren, sind bereits zahlreiche Methoden und Technologien entwickelt worden. Dabei wurde versucht, mehrere Arbeitsschritte mit einem einzigen Gerät durchzuführen und außerdem die zur Untersuchung erforderliche Blutmenge zu reduzieren. Letzteres erreicht man zum Beispiel, indem man den Durchmesser der Stechnadel verkleinert und die Einstichtiefe präzise einstellbar macht. Eine dünne Stechnadel bewirkt andererseits nur eine kleine Verletzung der Haut, so dass nur wenig oder gar kein Blut austritt. Durch zusätzliche Hilfsmaßnahmen, wie zum Beispiel Zusammendrücken oder periodische Anregung der Haut an der Einstichstelle, also Vibrationen, sowie durch die Sogwirkung eines erzeugten Unterdrucks lässt sich die Menge des austretenden Blutes vergrößern.

Die US 5 505 212 beschreibt eine Vorrichtung, mit der die Blutprobe in eine Kammer aufgenommen wird. Die Kammer besitzt eine flexible und kugelförmige obere Wand, in die eine Stechnadel eingelegt ist. Bei der Anwendung wird die Stechnadel durch die Wand nach unten in die Haut verschoben. Gleichzeitig wird die kugelförmige Wand ebenfalls nach unten gedrückt. Wenn der Druck von oben gelöst wird, kehrt die Vorrichtung zum Ausgangszustand zurück, wobei ein Unterdruck in der Kammer erzeugt wird, der die Blutaufnahme in die Kammer fördert. Die Sogwirkung eines Unterdrucks für das Ansaugen des Blutes ist schon seit Jahren bekannt, siehe hierzu beispielsweise die US 4 653 513. Der Nachteil der in der US 5 505 212 beschriebenen Vorrichtung ist, dass die Kammer und die kugelförmige obere Wand relativ groß bemessen sein müssen, um eine nennenswerte Sogwirkung erzeugen zu können. Infolgedessen wird relativ viel Blut angesaugt, mindestens mehrere Mikroliter, da die Kammer am Ende der Probenahme komplett mit Blut gefüllt ist, und eine geringere Füllmenge an Blut hier nicht einstellbar ist. Zu einer Antriebsmechanik und einer möglicherweise einsetzbaren Messeinrichtung wird kein Hinweis gegeben.

In der WO 02/100253 werden eine Vorrichtung und eine Methode beschrieben, mit deren Hilfe Blutproben in einen luftdichten Raum aufgenommen werden. Die Blutaufnahme wird durch Unterdruck begünstigt. Vom Funktionsprinzip her ist das Gerät für relativ große Probenmengen, das heißt 1 bis 5 Mikroliter, ausgelegt. Als weitere Möglichkeit wird die Integration einer Sensorschicht erwähnt.

In der DE 3708031 A1 wird eine in eine Kanüle integrierte Sensorschicht beschrieben. Die Kanüle mit der Sensorschicht wird in eine Saugglocke eingebaut, wo der Unterdruck durch die Heizung und anschließende Kühlung eines Reservoirs hervorgerufen wird. Die optische Messung wird aufwändig mit Hilfe von Lichtleiterfasern gelöst, wobei das Messgerät nicht in die Vorrichtung integriert ist.

Die US 2003/0055326 beschreibt eine Mikronadel für die Aufnahme von Körperflüssigkeiten und die Messung von Analyten. In die Mikronadel, das heißt eine Mikrokanüle mit einem Durchmesser von weniger als 350 Mikrometern, wird eine elektrochemische Sensoreinheit konzentrisch eingesetzt. Die Flüssigkeitsaufnahme wird durch Kapillarkräfte gefördert. Ein derartiges Konzept ist fehleranfällig. Beispielsweise liegt unter der Sensoreinheit in der Mikronadel ein geschlossener Raum, der nicht oder nur zum Teil durch Kapillarkräfte aufgefüllt werden kann, da stets eine Luftblase eingeschlossen bleibt.

Ein kompaktes Gerät wird in der US 4 637 403 beschrieben. Die Sensoreinheit sitzt auf dem Ende einer Stechnadel oder einer Kapillare auf. Die Auswertung erfolgt mit optischen Methoden. Zur Blutförderung wird ein Vakuum erzeugt. Ein Mikrocomputer übernimmt die Kontrolle und Logistik der Messprozedur und zeigt den Messwert an. Der Nachteil des Konzeptes ist der relativ lange Weg des Blutes bis zum Sensor.

Aus der US 2002/0198444 ist ebenfalls ein kompaktes Messgerät bekannt. Eine elektrische oder optische Sensoreinheit sowie ein Mikrocomputer für die Kontrolle und Logistik sind im Gerät integriert. Die Stechnadel und der bewegliche Teststreifen sind zwei getrennte Einheiten, die in die Lanzettenhalterung eingebaut sind. Das Blut wird hier nicht in das Gerät eingesaugt, sondern der Teststreifen wird zum Blutstropfen geführt. Wegen des komplexen Aufbaus der Lanzettenhalterung und Führung der beweglichen Teile ist die Herstellung dieses Einwegteils aufwändig. Da die Lanzettenhalterung nicht als Reservoir für das Blut vorgesehen ist, können außerdem leicht Funktionsfehler auftreten, zum Beispiel beim Verrutschen des Gerätes auf der Haut.

Seit langer Zeit sind Stechhilfen mit Vakuumunterstützung in verschiedensten Ausführungen bekannt, als Beispiele seien die US 4 653 513, EP 0 622 046, EP 0 838 195, US 6 332 871, US 6 086 545 und die EP 1 060 707 genannt. Die allgemeine Eigenschaft dieser Stechhilfen: Sie funktionieren mit Standardlanzetten oder dazu sehr ähnlichen Lanzetten, und die Integration eines Sensors oder eines Teststreifens ist nicht vorgesehen. In der US 6 332 871 ist lediglich die Möglichkeit erwähnt, dass ein Teststreifen seitlich zur Stechnadel ins Gerät eingeschoben werden kann. Ebenso ist bei diesen Stechhilfen der Raum zum Ansaugen relativ groß, und es muss eine vergleichsweise lange Kolbenbewegung realisiert werden, um ein effizientes Vakuum zu bilden.

In der WO 03/094752 und der WO 02/100254 werden die Einstechparameter der Stechnadel elektronisch eingestellt und die Lanzette mit elektromechanischen Bauteilen angesteuert. Es wird die Möglichkeit der Integration von Sensoren und Detektoren erwähnt, jedoch werden diese Lösungen nicht ausführlich dargestellt. Durch die Anwendung von elektronischen Hilfsmitteln könnte der Komfort erhöht werden. Damit verbunden sind jedoch hohe Herstellungskosten, eine aufwändige Energieversorgung und damit ein relativ großes und unhandliches Gerät.

Aus der letzten Zeit sind zahlreiche Erfindungen bekannt, bei denen Stechnadel und Sensor in eine Einheit integriert werden. Eine solche Einheit mit einem nicht näher spezifizierten Testelement ist aus der EP 1 287 785 bekannt, eine Einheit mit einem elektrischen Sensor beschreibt die US 6 607 658, und aus der EP 1 342 448 ist eine Einheit mit optischer Messung bekannt.

Kompaktgeräte mit integrierter Messeinheit sind Gegenstand der US 6 352 514, EP 1 362 551, EP 1 360 934, EP 1 360 933, WO 03/088834 und WO 02/101359. Diese Geräte nutzen die Kapillarkraft für die Förderung des Blutes zum Sensor. Mit Ausnahme der US 6 352 514 erfolgen Antrieb und Steuerung elektronisch. Obwohl die Handhabung durch die Elektronik einfach ist, sind die elektromechanischen Aktoren störanfälliger als rein mechanisch arbeitende Antriebseinrichtungen.

Die US 6 506 168 und US 6 306 104 beschreiben kompakte Messgeräte mit integrierter Stechhilfe, Sensoreinheit und Messsystem. Die Geräte sind vollelektronisch und verwenden eine Vakuumpumpe für die Förderung des Blutes. Der Nachteil in beiden Fällen ist, dass der Teststreifen und die Lanzette im Gerät getrennt vorliegen und einzeln ausgetauscht werden müssen. Diese umständliche Prozedur verschlechtert den Komfort und erhöht die Fehlerquellen und die Kontaminationsgefahr. Weiterhin verteuert der Einsatz von Pumpen für die Erzeugung eines Vakuums die Herstellungskosten und benötigt eine aufwändige Energieversorgung.

Die erwähnten Nachteile der Stechhilfen, Sensorsysteme und integrierten Geräte lassen sich wie folgt zusammenfassen: relativ große Mengen an benötigter Körperflüssigkeit, in der Regel ist das Blut, kein schmerzfreies Stechen, hohe Herstellungskosten, mangelnde Integration der einzelnen Komponenten und Verfahrensschritte, mangelhafte Hygiene und wenig Komfort, große und schwere Ausführungsformen durch hohen Energiebedarf.

Die Aufgabe der vorliegenden Erfindung besteht darin, eine Vorrichtung mit der die Probenahme einer Körperflüssigkeit, beispielsweise Blut, sowie die quantitative Analyse darin enthaltener Bestandteile durchgeführt werden kann, so zu verbessern, dass der Komfort für einen Benutzer bei geringen Kosten möglichst groß ist.

Die Aufgabe wird erfindungsgemäß gelöst durch eine Vorrichtung nach dem Anspruch 1.

Die erfindungsgemäße Vorrichtung zur Probenahme und Analyse von Körperflüssigkeiten, dabei ist insbesondere an Blut gedacht, umfasst ein Handteil, das eine pneumatische Antriebseinrichtung aufnimmt, und eine Sensoreinheit, die eine Mikronadel, die fest mit einer elastischen Membran verbunden ist, und mehrere Sensorsysteme enthält. Das Handteil und die Sensoreinheit sind lösbar miteinander verbunden, und die Antriebseinrichtung steht in Wechselwirkung mit der Sensoreinheit auf die Weise, dass die pneumatische Antriebseinrichtung über einen komprimierten Luftraum einen gewissen Druck auf die elastische Membran zum Einstechen der Mikronadel auf eine Hautoberfläche für die Entnahme von Körperflüssigkeit ausübt. Anschließend wird der Druck auf die elastische Membran verringert. Durch das Zurückweichen der Membran in ihre ursprüngliche Form wird im Inneren der Sensoreinheit ein Unterdruck erzeugt und Körperflüssigkeit aus der Einstichstelle zu mindestens einem Sensorsystem transportiert. Im Sensorsystem wird durch die zu bestimmenden Bestandteile der Körperflüssigkeit eine physikalische oder chemische Eigenschaftsänderung des Sensorsystems bewirkt. Weiterhin umfaßt die Sensoreinheit einen drehbar einrastenden Zuführungskanal für die Körperflüssigkeit, so dass Körperflüssigkeit jeweils nur auf ein Sensorsystem geleitet wird und nach erfolgter Messung eines Analyten der Zuführungskanal um eine Rasterstellung weiter drehbar ist, um den Zugang von Körperflüssigkeit zum nächsten Sensorsystem zu öffnen.

Die erfindungsgemäße Vorrichtung hat zum Vorteil, dass sie vergleichsweise günstig in der Herstellung ist. Die pneumatische Antriebseinrichtung zum Einstechen der Mikronadel und anschließenden Ansaugen von Körperflüssigkeit beruht auf einem einfachen mechanischen Prinzip, ist leicht zu handhaben und wenig störanfällig. Mit einer entsprechenden Bemessung des Innenraums der Sensoreinheit kann die erforderliche Probemenge an Körperflüssigkeit optimal reduziert werden. Die Verwendung einer Mikronadel mit Unterstützung einer Sogwirkung zur Förderung des Blutaustritts halten die Schmerzen dabei gering. Besonders günstig ist die lösbare Verbindung von Handteil und Sensoreinheit, denn dadurch ist es möglich, eine entsprechende Sensoreinheit als sterilen Wegwerfartikel auszuführen, der beispielsweise als Spritzgussteil kostengünstig zu fertigen ist.

Vorteilhafte Ausführungsformen der Vorrichtung sind Gegenstand der Unteransprüche.

Eine bevorzugte Ausgestaltung der Vorrichtung besteht darin, dass das Handteil eine Messeinheit zur Bestimmung der im Sensorsystem durch Einwirken der zu bestimmenden Bestandteile der Körperflüssigkeit hervorgerufenen physikalischen oder chemischen Eigenschaftsänderungen enthält. Die Messeinheit ermittelt aus diesen Eigenschaftsänderungen den Konzentrationswert eines zu bestimmenden Bestandteils. Vorteilhafterweise erfolgt eine Anzeige auf einem in die Vorrichtung integrierten Display. Auf diese Weise ist es möglich, in einem einzigen Prozess eine Probennahme durchzuführen, den gewünschten Analysewert zu bestimmen und für den Benutzer unmittelbar anzuzeigen. Ebenso ist die Speicherung von Messwerten möglich. In einer speziellen Ausführungsform kann das Ergebnis auch mit einem Transponder oder einem ähnlichen Hilfsmittel an ein zweites Gerät weitergegeben werden. Dieses zweite Gerät kann beispielsweise eine automatische Spritze oder eine Pumpe zur Verabreichung entsprechender Therapeutika wie Insulin sein. Es kann sich hierbei auch um ein Anzeigegerät wie ein Mobiltelefon, eine Armbanduhr, einen PC oder einen PDA handeln.

Da eine Analyse von Körperflüssigkeiten kostengünstig, zuverlässig und schnell erfolgen soll, sind optische Messverfahren für das Sensorsystem, insbesondere fluoreszenzspektroskopische Messverfahren, von besonderem Vorteil. Bei der Fluoreszenzmessung kann eine minimale Fläche an Sensorsystem verwendet werden, wodurch die Produktion der Sensoreinheiten kostengünstig wird. In einer bevorzugten Ausführung wird als Nachweismethode die FRET-Methode (Fluorescence Resonance Energy Transfer) verwendet.

Die quantitative Auswertung der im Sensorsystem erfolgenden biochemischen Reaktion kann mit optischen Methoden wie UV/Vis-Absorptionsmessung, SPR (Surface Plasmon Resonance), IR-Spektroskopie, Ellipsometrie, Colorimetrie, Fluoreszenzspektrometrie oder auch durch elektrochemische oder sonstige Bestimmungsmethoden erfolgen. Als elektrochemische Messmethoden kommen die Amperometrie und die Coulometrie in Betracht. Andere Methoden arbeiten mit Hilfe von SAW (Surface Acustic Waves), Schwingquarzen, Impedanzmessungen und Cantilevern.

Die erfindungsgemäße Vorrichtung ermöglicht die simultane quantitative Bestimmung mehrerer Bestandteile der Körperflüssigkeit mit demselben oder mit unterschiedlichen Messverfahren. Dazu werden in der Sensoreinheit mehrere Sensorsysteme untergebracht. Auch wenn hierbei eine etwas höhere Blutmenge benötigt wird, ist durch die räumlich enge Verbindung von Entnahmestelle und Sensorsystemen der Blutverbrauch wesentlich kleiner als bei herkömmlichen Methoden, bei denen eine wesentlich größere Blutmenge entnommen und zu räumlich getrennten Messgeräten gebracht werden muss. Bei der Fluoreszenzmessung, insbesondere mit der FRET-Methode, ist es sogar möglich, mehrere Analyten auf derselben Fläche eines Sensorsystems zu messen, da hier durch Messungen bei unterschiedlichen Anregungswellenlängen die verschiedenen Nachweissysteme gemischt auf einem Sensorsystem untergebracht werden können.

Die lösbare Verbindung zwischen dem Handteil und der Sensoreinheit ist in vorteilhafter Weise als schnell einrastende Verbindung ausgestaltet. Das kann beispielsweise eine Steck-, Klemm-, Schnapp-, Schraub- oder Klebeverbindung sein. Wichtig ist dabei in jedem Fall, dass das Lösen und Herstellen der Verbindung schnell und einfach erfolgt und ein Verbinden von Handteil und Sensoreinheit zu einem stabilen Gerät führt.

In einer weiteren Ausführungsform weist die Sensoreinheit eine Kontrolleinrichtung auf, um die korrekte, insbesondere ausreichende Befüllung, mit Körperflüssigkeit nachzuweisen. So ist es für eine zuverlässige Analyse erforderlich, dass auf jeden Fall eine genügende Menge an Körperflüssigkeit zur Verfügung steht. Die entsprechende Kontrolleinrichtung hierfür kann eine elektrische Widerstandsmesseinheit mit piezoelektrischen oder elektromagnetischen Elementen zur Stromversorgung umfassen. Als ausreichende Befüllung für eine zuverlässige Analyse sind beispielsweise 0.5 µl oder weniger anzusehen. Diese Menge kann der Kontrolleinrichtung als Sollwert vorgegeben sein.

In einer weiteren Ausführungsform ist vorgesehen, dass das Handteil ein erstes Einstellglied für eine variable Vorgabe der Einstichtiefe der Mikronadel aufweist. Das macht sich besonders dann vorteilhaft bemerkbar, wenn die Einstichtiefe individuell an die Einstichstelle auf der Haut angepasst werden soll. So hängt es stark vom Ort des Einstichs ab, ob die Hautoberfläche dort dicker oder dünner ist und ob die Blutgefäße tiefer oder weniger tief liegen.

Darüber hinaus ist es möglich, dass ein zweites Einstellglied am Handteil für eine variable Vorgabe der Einstechgeschwindigkeit angebracht ist. Auf diese Weise ist eine individuelle Anpassung möglich, um den Schmerz möglichst gering zu halten. Eine entsprechende Möglichkeit besteht für die variable Vorgabe der Ansauggeschwindigkeit von Körperflüssigkeit durch die Mikronadel mittels eines dritten Einstellglieds.

Für eine Messung der Körperflüssigkeit durch das Sensorsystem kann es vorteilhaft sein, eine Filteranordnung vorzuschalten. Eventuell ist es auch erforderlich, die Körperflüssigkeit vorher besonders aufzubereiten. Das kann geschehen durch Reagenzien und/oder Spüllösungen aus Vorratsbehältern, die ebenfalls vor das Sensorsystem geschaltet werden können und eine Körperflüssigkeit für die Probenahme entsprechend aufbereiten.

Das Verfahren zur Probenahme und Analyse von Körperflüssigkeiten besteht aus mehreren Schritten. In einem ersten Schritt übt eine von einem Handteil aufgenommene pneumatische Antriebseinrichtung über einen komprimierten Luftraum Druck auf eine elastische Membran in einer Sensoreinheit zum Einstechen einer an der elastischen Membran befestigten Mikronadel auf eine Hautoberfläche aus, und die Mikronadel dringt in die Hautoberfläche ein. Anschließend wird der Druck auf die elastische Membran verringert. Durch das Zurückweichen der Membran in die ursprüngliche Form wird im Inneren der Sensoreinheit ein Unterdruck erzeugt und Körperflüssigkeit aus der Einstichstelle dadurch zu mindestens einem Sensorsystem transportiert. Im Sensorsystem wird durch die zu bestimmenden Bestandteile der Körperflüssigkeit eine physikalische oder chemische Eigenschaftsänderung des Sensorsystems bewirkt.

In einem nächsten Schritt bestimmt die Messeinheit aus diesen Eigenschaftsänderungen einen Konzentrationswert des zu bestimmenden Bestandteils und zeigt ihn ggf. auf einem Display an.

Für ein solches Verfahren wird mit Vorteil eine erfindungsgemäße Vorrichtung verwendet.

Die Erfindung wird anhand der folgenden Zeichnungen im Einzelnen erläutert.

Es zeigen:
- Fig. 1: eine schematische Darstellung einer erfindungsgemäßen Vorrichtung in einem Längsschnitt,
- Fig. 2a bis 2h: eine Probenahme und Analyse von Körperflüssigkeiten mit der Vorrichtung aus Fig. 1 in acht zeitlich aufeinander folgenden Phasen,
- Fig. 3: eine perspektivische Ansicht auf eine erfindungsgemäße Vorrichtung zur Probenahme und Analyse von Körperflüssigkeiten,
- Fig. 4: eine Darstellung der Vorrichtung zur Probenahme und Analyse von Körperflüssigkeiten mit aufgeschnittenem Handteil,
- Fig. 5: den unteren Bereich des Handteils mit abgenommener Sensoreinheit,
- Fig. 6: eine schematische Darstellung einer Sensoreinheit im Längsschnitt,
- Fig. 7: eine weitere Darstellung der Vorrichtung mit aufgeschnittenem Handteil und eine detaillierte Wiedergabe des Auslösemechanismus,
- Fig. 8: eine Vorrichtung zur Probenahme und Analyse von Körperflüssigkeiten, die mit optischen Messverfahren arbeitet,
- Fig. 9: eine vergrößerte Ansicht der Sensoreinheit der Vorrichtung aus Fig. 8,
- Fig. 10: eine weitere Detailansicht der Sensoreinheit bei Anwendung eines optischen Messverfahrens,
- Fig. 11a bis 11e: mehrere schematische Darstellungen von Sensoreinheiten im Längsschnitt mit verschiedenen Ausgestaltungen der elastischen Membran,
- Fig. 12: eine Draufsicht auf eine Sensoreinheit mit verschiedenen Sensorsystemen,
- Fig. 13: eine schematische Darstellung einer Sensoreinheit für den mehrmaligen Gebrauch im Schnitt,
- Fig. 14: eine schematische Darstellung einer Sensoreinheit im Längsschnitt mit einer zusätzlichen Filteranordnung und Vorratsbehältern.

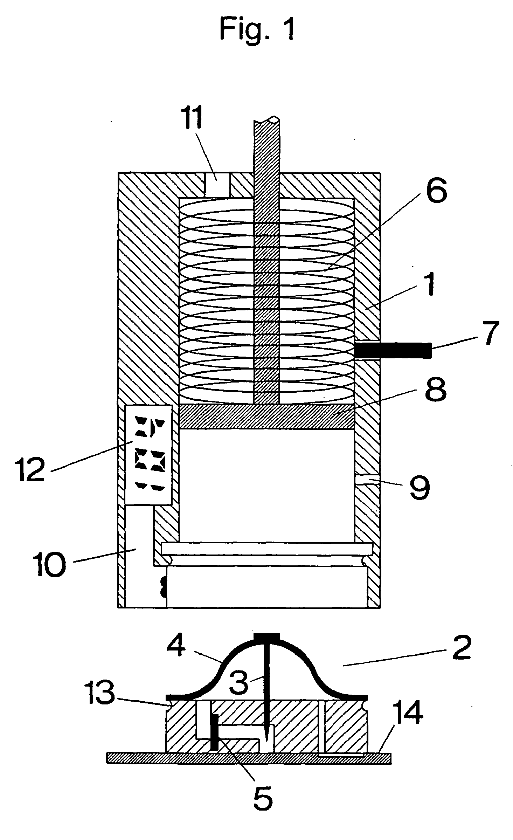

Fig. 1 stellt eine erfindungsgemäße Vorrichtung im Längsschnitt dar. Die Vorrichtung besteht aus einem wieder verwendbaren Handteil 1 und einer Sensoreinheit 2, die als Wegwerfartikel ausgebildet ist. In dem Handteil 1 ist eine Antriebseinrichtung untergebracht, die aus einem Kolben 8 besteht, der durch die Kraft einer Feder 6 in dem Gehäuse des Handteils 1 nach unten getrieben wird. Eine untere Druckausgleichsöffnung 9 und eine obere Druckausgleichsöffnung 11 sorgen für ein ungehindertes Vorschnellen der Antriebseinrichtung. Die Antriebseinrichtung wird betätigt durch einen Auslöser 7, der von außen am Handteil 1 vom Benutzer eingedrückt werden kann. Des Weiteren sind in dem Handteil 1 eine Messeinheit 10 sowie ein Display 12 angeordnet. Die Sensoreinheit 2 umfasst eine Mikronadel 3, welche an einer elastischen Membran 4 befestigt ist, so dass bei Druck auf die Membran 4 die Mikronadel 3 in die Oberfläche einer Haut getrieben werden kann. Die Sensoreinheit 2 und das Handteil 1 werden über einen Schnappverschluss 13 miteinander in Verbindung gebracht. Nach einer bestimmungsgemäßen Benutzung der Vorrichtung wird die Sensoreinheit 2 weggeworfen, und das Handteil 1 wird wieder verwendet. Aus diesem Grund müssen Sensoreinheit 2 und Handteil 1 voneinander getrennt werden, was mit Hilfe einer eigens dafür vorgesehenen Abwurfkante 14 erfolgt. Die pneumatische Antriebseinrichtung und die Sensoreinheit 2 stehen nun in der Weise miteinander in Wechselwirkung, dass durch Herunterfahren des Kolbens 8 über einen komprimierten Luftraum die elastische Membran 4 sowie die daran befestigte Mikronadel 3 nach unten in die Hautoberfläche gedrückt werden. Durch die untere Druckausgleichsöffnung 9 kann anschließend der Überdruck entweichen. Dadurch lässt der Druck auf die elastische Membran 4 nach, sie kehrt in ihre Ausgangsform zurück, und die Mikronadel 3 bewegt sich wieder nach oben, wobei in der Sensoreinheit 2 Unterdruck entsteht. Dadurch wird Körperflüssigkeit aus der Einstichstelle angesaugt, und die Körperflüssigkeit wird dem Sensorsystem 5 zugeführt. Im Sensorsystem 5 wird durch die zu bestimmenden Bestandteile der Körperflüssigkeit eine physikalische oder chemische Eigenschaftsänderung des Sensorsystems 5 bewirkt. Die Messeinheit 10 bestimmt aus diesen Eigenschaftsänderungen einen Konzentrationswert der zu bestimmenden Bestandteile, und sie werden auf dem Display 12 angezeigt.

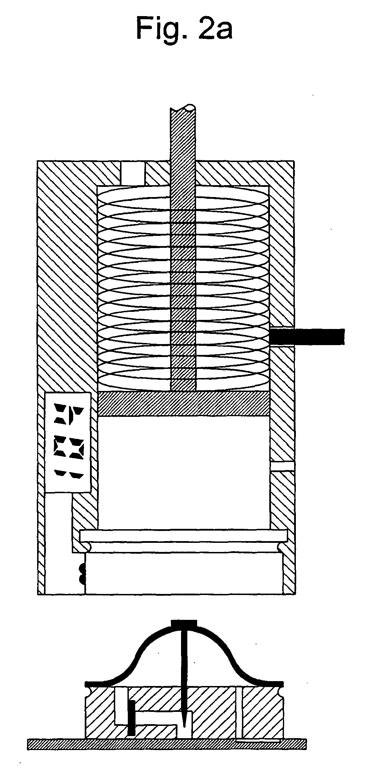

Eine Probenahme und anschließende Analyse von Körperflüssigkeit mit der Vorrichtung aus Fig. 1 wird anhand der acht Fig. 2a bis 2h veranschaulicht. Die Fig. 2a bis 2h stellen zeitlich aufeinander folgend verschiedene Phasen der Probenahme und Analyse dar. Die Fig. 2a bis 2h sind identische Wiedergaben der Vorrichtung aus Fig. 1. Auf Bezugszeichen wird deshalb verzichtet.

Die Fig. 2a zeigt den Ausgangszustand der Vorrichtung mit dem wieder verwendbaren Handteil 1 und der als Wegwerfartikel ausgebildeten Sensoreinheit 2, die vor Gebrauch vorzugsweise in einer sterilen Verpackung aufbewahrt wird.

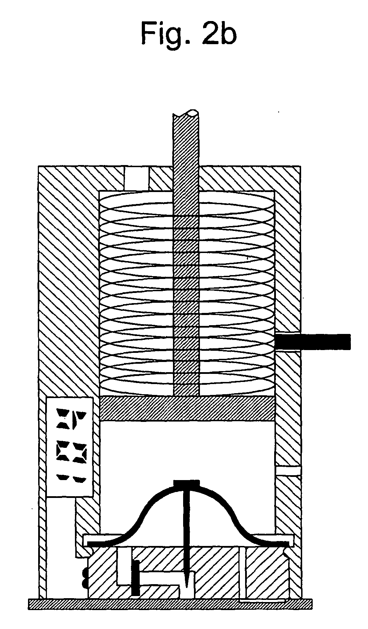

Die Fig. 2b zeigt die Sensoreinheit 2, die mittels des eingeschnappten Schnappverschlusses 13 mit dem Handteil 1 verbunden ist.

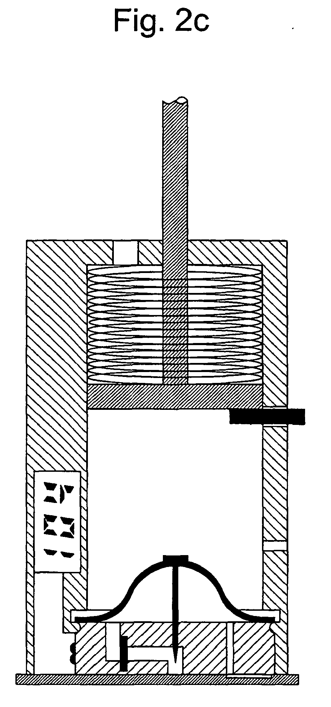

In der Fig. 2c ist erkennbar, dass die Feder 6 im Handteil 1 gespannt wird, d.h., der Kolben 8 befindet sich in seiner oberen Position. Der am Umfang des Handteils 1 befindliche Auslöser 7 hält die Feder 6 davon ab, sich zu entspannen und den Kolben 8 nach unten voranzutreiben.

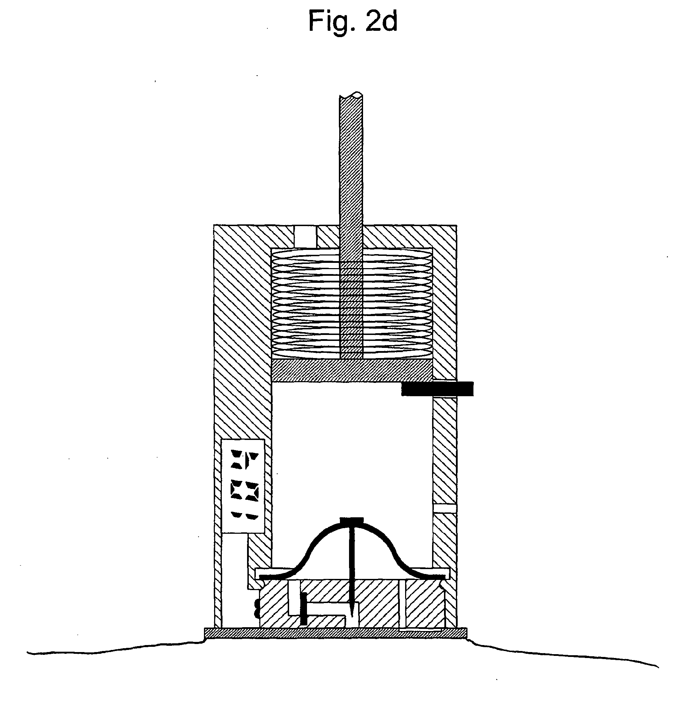

Die Fig. 2d stellt dar, wie die im Handteil 1 eingesetzte Sensoreinheit 2 auf eine Hautoberfläche aufgesetzt wird.

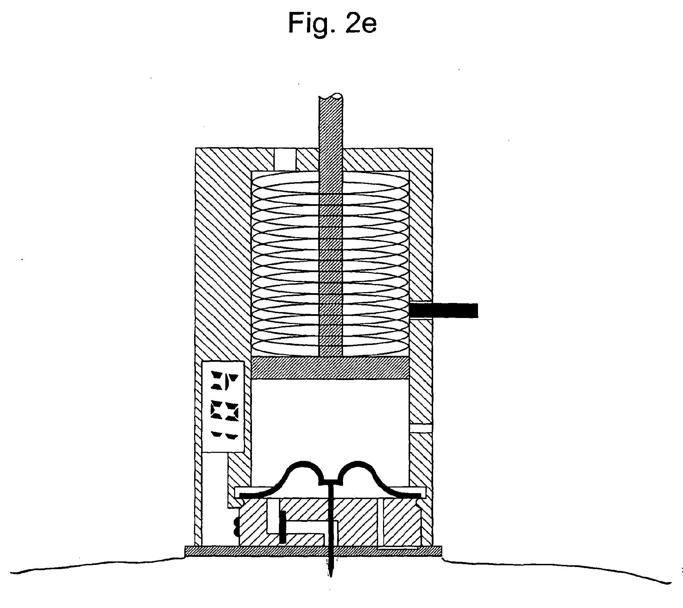

Die Fig. 2e stellt eine sog. Stechphase dar. Durch Betätigung des Auslösers 7 wird die Feder 6 entspannt, und der Kolben 8 bewegt sich nach unten. Zwischen dem Kolben 8 und der elastischen Membran 4 wird durch die schnelle Bewegung des Kolbens 8 ein komprimierter Luftraum aufgebaut. Im Handteil 1 kann Luft durch die obere Druckausgleichsöffnung 11 eindringen. Die elastische Membran 4 verformt sich, und die Mikronadel 3 sticht durch die Hautoberfläche. Die Luft innerhalb der Sensoreinheit 2 wird durch seitliche Schlitze nach außen geleitet. Diese Schlitze können als Öffnungen mit beliebigem Querschnitt ausgeführt sein und Schließelemente enthalten, die eine Ventilfunktion aufweisen und sich beispielsweise erst nach Überschreiten einer vorgegebenen Druckdifferenz öffnen.

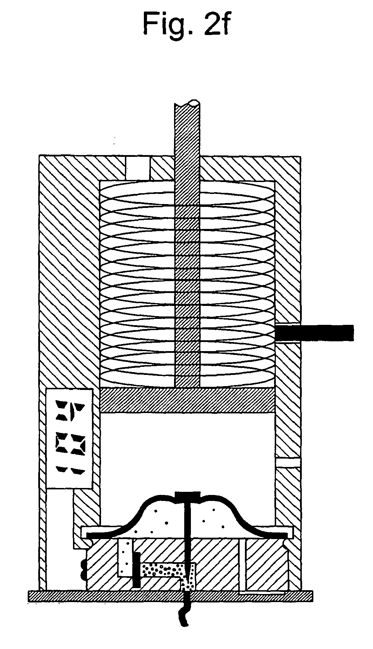

In der Fig. 2f ist die sog. Saugphase dargestellt. Durch die untere Druckausgleichsöffnung 9 entweicht komprimierte Luft. Nach Erreichen der maximalen Einstichtiefe entspannt sich die elastische Membran 4 wieder und bewegt sich in den Ausgangszustand zurück. Dabei wird ein Unterdruck innerhalb der Sensoreinheit 2 aufgebaut und Körperflüssigkeit zu dem Sensorsystem 5 geleitet. Eine chemische, biochemische oder physikalische Reaktion findet an dem Sensorsystem 5 statt. Die Folge davon ist eine physikalische oder chemische Eigenschaftsänderung im Sensorsystem 5.

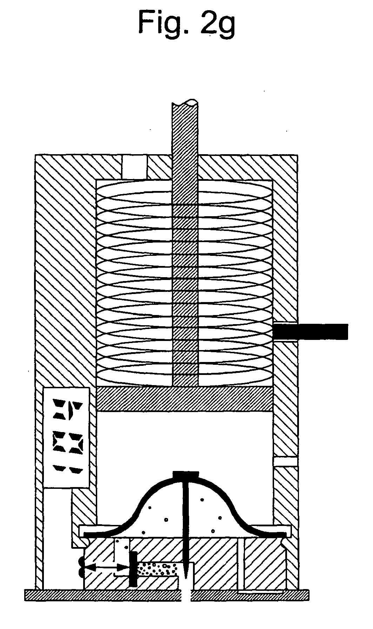

Die Fig. 2g zeigt die eigentliche Messphase. Die im Sensorsystem 5 hervorgerufenen physikalischen oder chemischen Eigenschaftsänderungen werden durch die Messeinheit 10 bestimmt und anschließend die ausgewerteten Messwerte im Display 12 angezeigt.

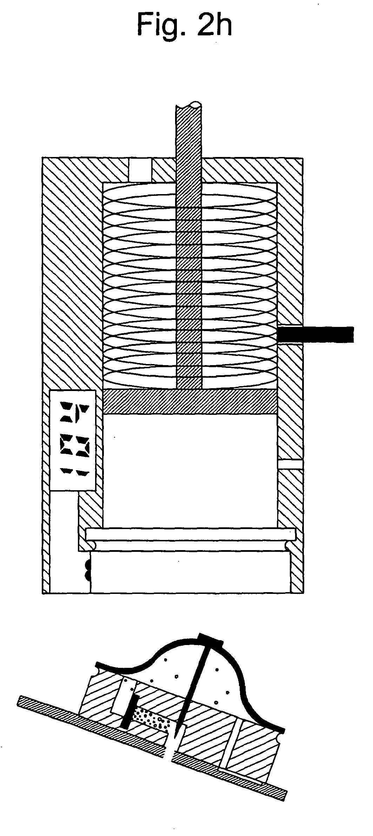

Nach Beendigung der Probenahme und Analyse wird die Sensoreinheit 2 entfernt, was in der Fig. 2h dargestellt ist. Eine Hilfe beim Abtrennen der Sensoreinheit 2 vom Handteil 1 stellt die Abwurfkante 14 dar. Das Handteil 1 ist danach durch Aufstecken einer weiteren Sensoreinheit 2 für einen neuen Einsatz bereit.

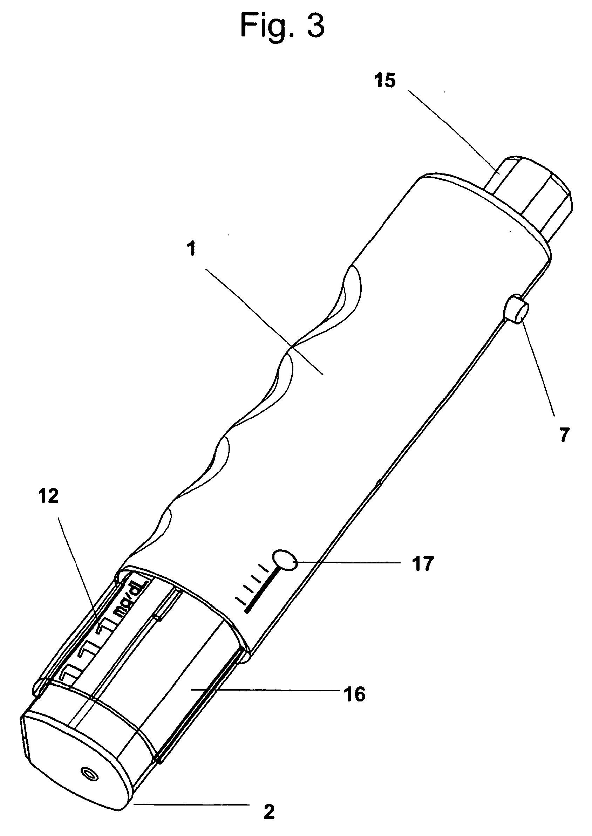

Die Fig. 3 stellt eine perspektivische Ansicht auf eine erfindungsgemäße Vorrichtung zur Probenahme und Analyse von Körperflüssigkeiten dar. Das Handteil 1 enthält einen Mechanismus zum pneumatischen Antrieb mit Gewindespindel 18 und Feder, die hier nicht erkennbar sind. Durch Drehen eines ersten Einstellglieds 15, das ist hier ein Drehknopf, wird die pneumatische Antriebseinrichtung gespannt. Durch Drücken des Auslösers 7 kann dann anschließend die pneumatische Antriebseinrichtung ausgelöst werden, und es entsteht ein komprimierter Gasraum im Inneren des Handteils 1. Dadurch wird Kraft auf die Sensoreinheit 2 ausgeübt. Je nachdem, wie weit der Drehknopf 15 gedreht wird, spannt sich die Feder an der Gewindespindel 18 in der pneumatischen Antriebseinrichtung unterschiedlich stark. Damit kann die Einstichtiefe variabel gestaltet werden. Durch ein zweites Einstellglied 17 kann die Einstechgeschwindigkeit eingestellt werden. Außerdem ist eine Messeinheit 10, die hier nicht sichtbar ist, in das Handteil integriert, die physikalische oder chemische Eigenschaftsänderungen im Sensorsystem 5 bestimmen kann. Die bestimmten Werte werden anschließend vom Display 12 angezeigt. Beim Spannen der pneumatischen Antriebseinrichtung kann elektrische Energie durch ein entsprechendes Piezoelement gewonnen werden. Die Erzeugung von elektrischem Strom kann aber auch elektromagnetisch durch einen kleinen Magneten erfolgen, was den Vorteil hat, dass dann die Phase des Entspannens der Feder an der Gewindespindel 18 ausgenutzt werden kann. Vorzugsweise wird die elektrische Energie auf die als zweite genannte Weise gewonnen. Die gewonnene elektrische Energie kann dann zum Beispiel für eine Kontrolleinrichtung zum Nachweis der korrekten Befüllung mit Körperflüssigkeit oder für die Energieversorgung von autarken Sensoren verwendet werden. In der Fig. 3 ist außerdem eine Hülse 16 zum Abwurf der Sensoreinheit 2 nach Gebrauch dargestellt.

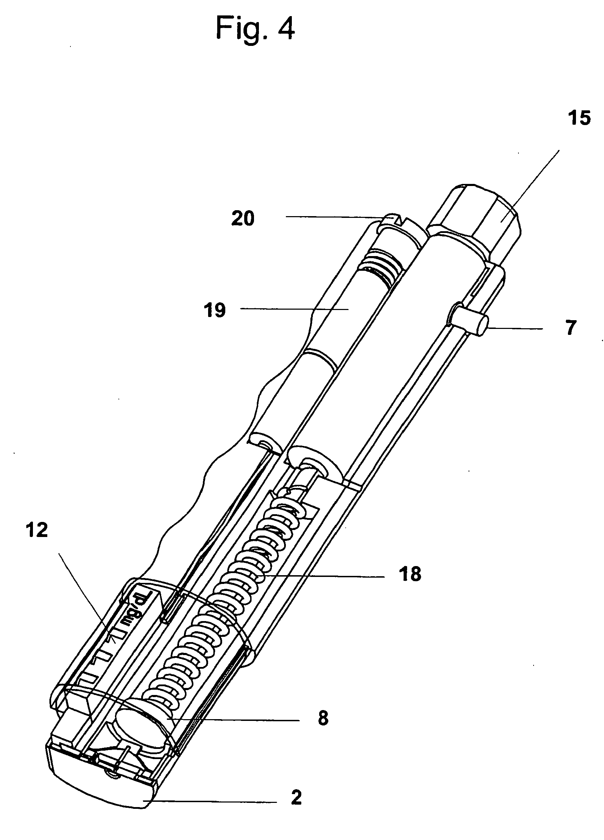

Die Fig. 4 gibt eine Ansicht ins Innere der Vorrichtung zur Probenahme und Analyse von Körperflüssigkeiten frei. Die Messeinheit 10 ist im Handteil 1 integriert, und die Sensoreinheit 2 ist aufgesetzt. Die im Inneren des Handteils 1 liegende Gewindespindel 18 mit der Feder treibt nach Betätigen des Auslösers 7 den Kolben 8 nach unten, drückt dabei die über der elastischen Membran 4 in der Sensoreinheit 2 befindliche Luftsäule zusammen und treibt damit die in die Sensoreinheit 2 integrierte Mikronadel 3 nach unten. Die Energieversorgung wird hier über Batterien 19 in einem eigens dafür angebrachten Batteriefach 20 gewährleistet.

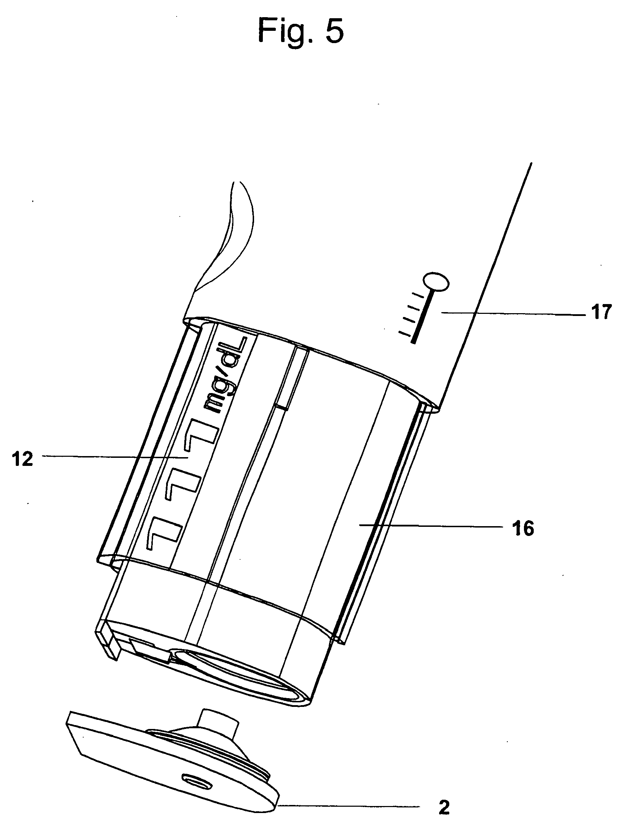

Die Fig. 5 stellt den unteren Bereich des Handteils 1 mit abgenommener Sensoreinheit 2 dar. Sie war über einen hier nicht dargestellten Schnappverschluss 13 am Handteil 1 befestigt und wurde anschließend mittels 13 am Handteil 1 befestigt und wurde anschließend mittels Druck durch die Hülse 16 auf die Abwurfkante 14 vom Handteil 1 getrennt. Die Sensoreinheit 2 ist üblicherweise für den einmaligen Gebrauch bestimmt.

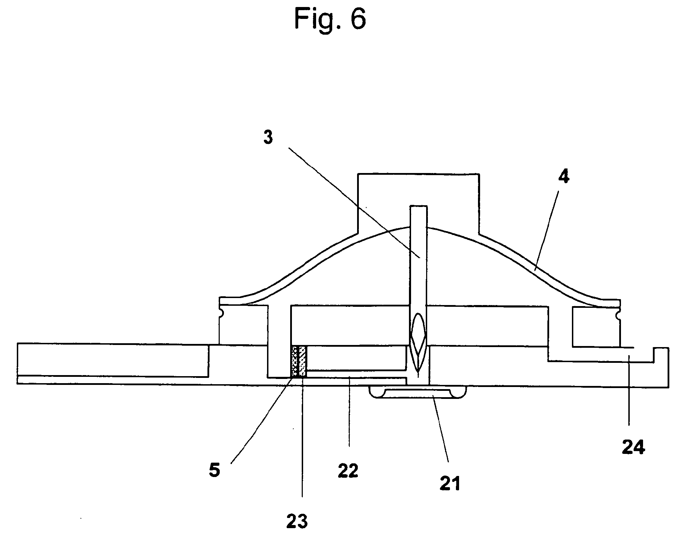

Die Fig. 6 zeigt eine schematische Darstellung der Sensoreinheit 2 im Längsschnitt. Die in der elastischen Membran 4 befestigte Mikronadel 3 bewegt sich bei Einwirkungen von äußerem Druck auf die elastische Membran 4 nach unten und dringt in die Hautoberfläche ein, sofern das Handteil 1 mit der Sensoreinheit 2 vorher auf die Hautoberfläche aufgesetzt wurde. Dabei entweicht Luft, wobei zum Beispiel ein Entlüftungskanal 24 wie ein Ventil ausgeführt sein kann, das ein Durchdringen von Luft nur in eine Richtung, nämlich von innen nach außen, zulässt. Die Mikronadel 3 zieht sich beim Nachlassen des äußeren Drucks auf die elastische Membran 4 wieder zurück, wobei gleichzeitig in der Sensoreinheit 2 ein Unterdruck entsteht, welcher Körperflüssigkeit in die Sensoreinheit 2 zieht. Durch eine Kapillare 22 gelangt diese direkt auf eine Filteranordnung 23, wird gefiltert und kommt danach mit dem Sensorsystem 5 in Berührung. Ein Abdichtring 21 dient zur Abdichtung des Sensorsystems 5 gegenüber der Hautoberfläche. In einer speziellen Ausführungsform kann der Abdichtring 21 mit einer Klebeschicht versehen sein, um einen besseren Kontakt mit der Haut zu gewährleisten. Die Unterseite der Sensoreinheit 2 kann durch eine abziehbare Schutzfolie, die hier bereits entfernt wurde, bis zur Verwendung steril gehalten werden. Bei einer anderen Ausführungsform kann die Schutzfolie beim Stechen auf der Unterseite der Sensoreinheit 2 verbleiben, muss dann aber vorgespannt sein, damit beim Durchstechen eine ausreichend große Öffnung entsteht.

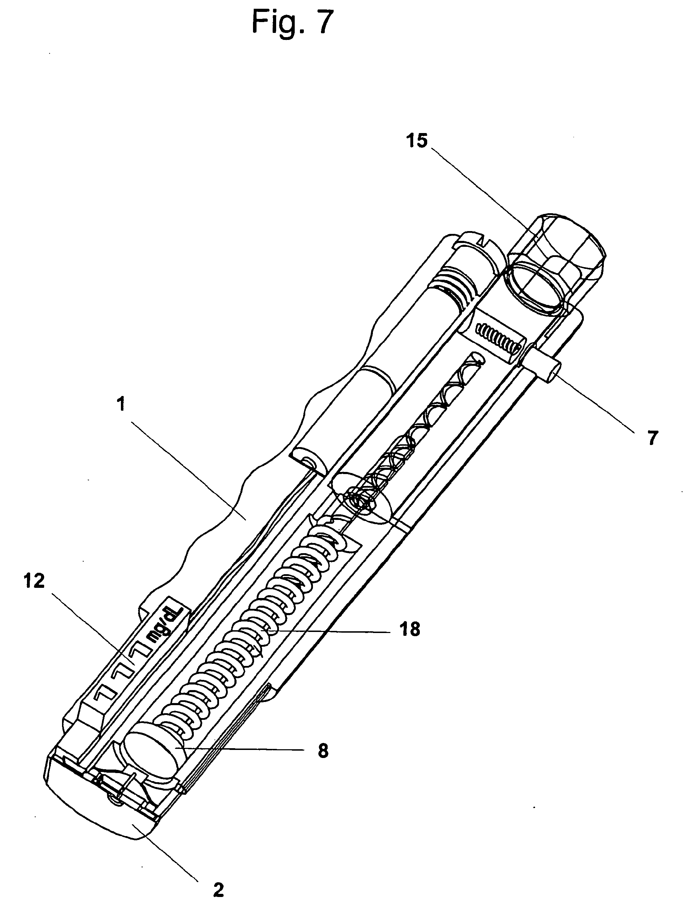

Die Fig. 7 stellt eine weitere Vorrichtung mit aufgeschnittenem Handteil 1 und einer detaillierten Wiedergabe des Auslösemechanismus dar. Durch Drücken des Auslösers 7 wird die Gewindespindel 18 mit Feder freigegeben und treibt den Kolben 8 nach unten.

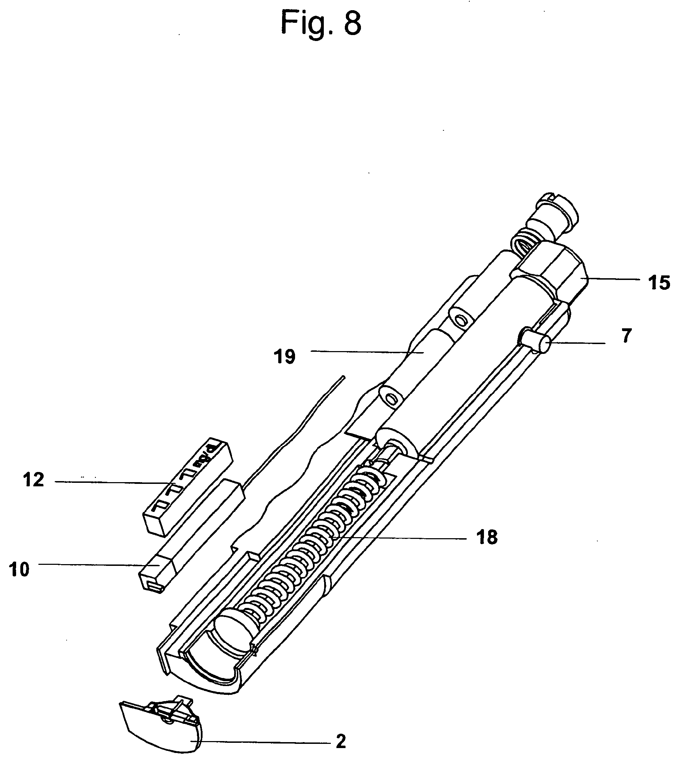

In der Fig. 8 ist eine Vorrichtung zur Probenahme und Analyse von Körperflüssigkeiten zu sehen, die mit optischen Messverfahren arbeitet. Insbesondere werden hier Methoden der Fluoreszenzspektrometrie verwendet. Die Messeinheit 10 beinhaltet eine Lichtquelle 27 und eine Photodiode 26 sowie die erforderliche Elektronik. Die Stromversorgung erfolgt mit Hilfe einer Batterie 19. An der Außenseite des Handteils 1 befindet sich ein Display 12 zur Anzeige der gewonnenen Messwerte.

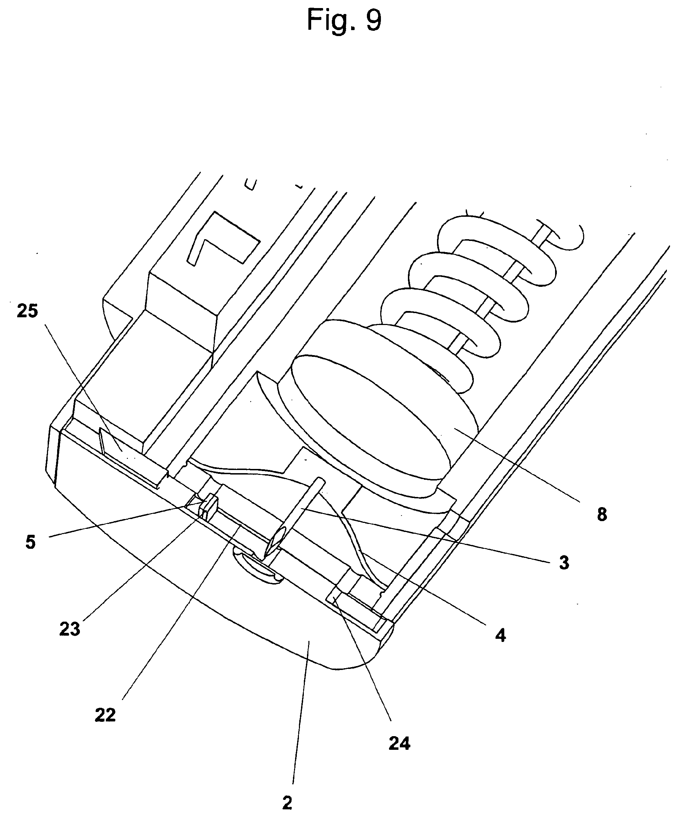

Die Fig. 9 ist eine vergrößerte Ansicht der Sensoreinheit 2 der Vorrichtung aus Fig. 8. Ein Lichtleiterblock 25 befindet sich an der Unterseite des Handteils 1 und ist fest mit diesem verbunden. Er leitet das Anregungslicht, welches aus der Lichtquelle 27 stammt, direkt auf das Sensorsystem 5, fängt das emittierte Licht wieder auf und leitet es zu der in der Messeinheit 10 befindlichen Photodiode 26, die hier nicht abgebildet ist.

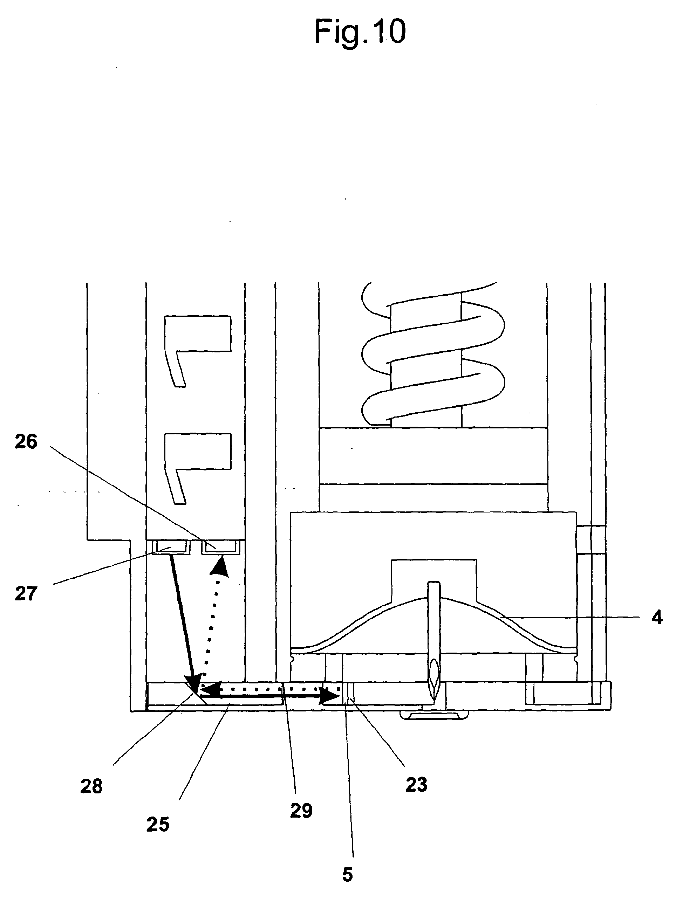

Die Fig. 10 zeigt eine weitere Detailansicht der Sensoreinheit 2 bei Anwendung eines optischen Messverfahrens. Das Anregungslicht tritt aus der Lichtquelle 27 aus und wird über Totalreflektion an einer Oberfläche 28 über eine Linse 29 auf das Sensorsystem 5 geführt. Das vom Sensorsystem 5 emittierte Licht wird über die Linse 29 und die total reflektierende Oberfläche 28 zu der Photodiode 26 geleitet. Die gesamte optische Anordnung ist vorzugsweise als integriertes optisches Bauteil ausgeführt.

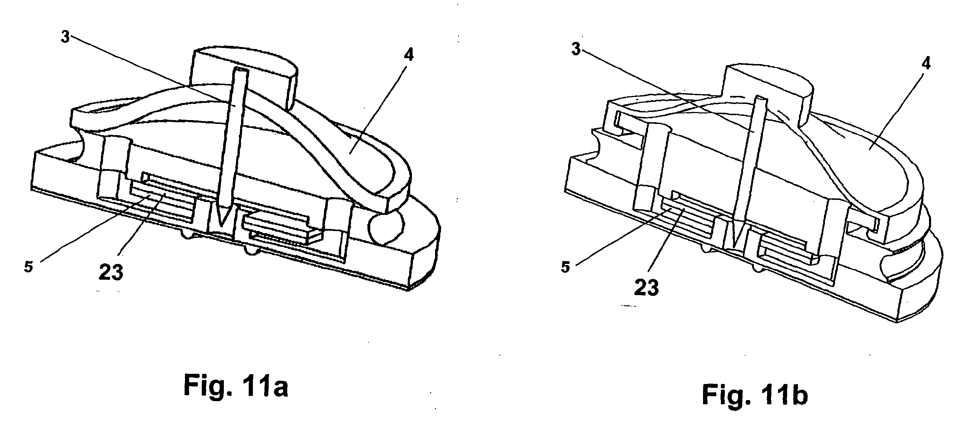

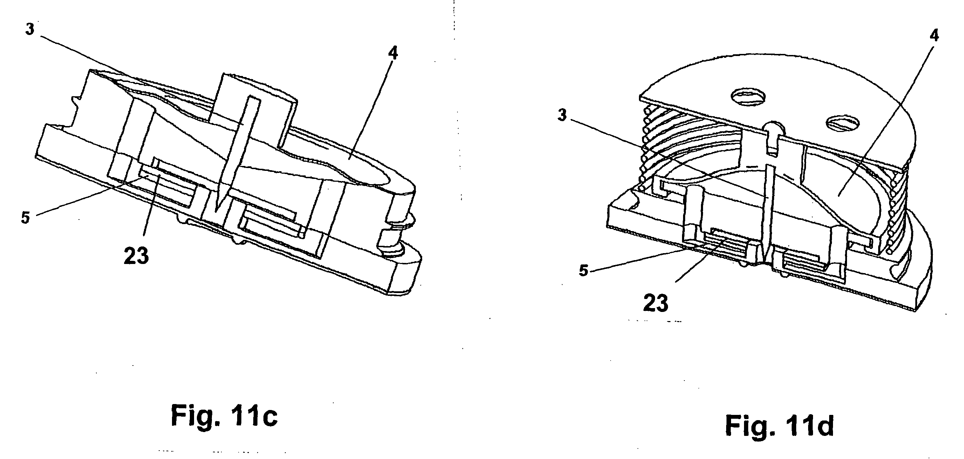

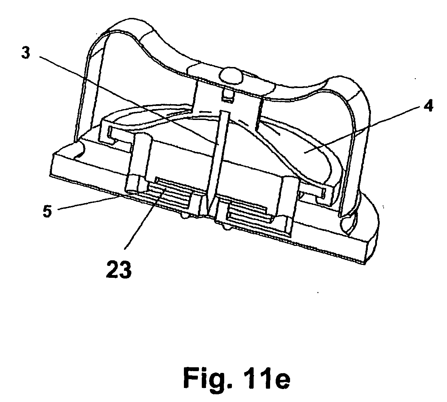

Die Fig. 11a bis 11e zeigen schematische Darstellungen einer Sensoreinheit 2 im Längsschnitt mit verschiedenen Ausgestaltungen der elastischen Membran 4. In allen Fällen dringt die an der elastischen Membran 4 befestigte Mikronadel 3 bei Einwirkung von äußerem Druck auf die elastische Membran 4 in die Hautoberfläche ein. Die Fig. 11b zeigt eine Ausführung einer Sensoreinheit 2, bei der die elastische Membran 4 in ein Spritzgussteil eingerastet ist. Bei Ausübung von äußerem Druck auf die elastische Membran 4 entweicht die Luft aus der Sensoreinheit 2 seitlich durch die Einrastung. Bei Entspannung der elastischen Membran 4 wird in der Sensoreinheit 2 ein Unterdruck aufgebaut, der die Körperflüssigkeit aus der Haut saugt. Eine weitere Ausführungsform ist in der Fig. 11c dargestellt, bei der eine ebene elastische Membran 4 und eine entsprechende Vertiefung verwendet werden, die eine Variation des Volumens analog zur elastischen Membran 4 der anderen Sensoreinheiten 2 ermöglichen. Die elastische Membran 4 kann alternativ durch eine Feder oder einen Bügel in Form gehalten werden, wie es in den Fig. 11d und 11e dargestellt ist.

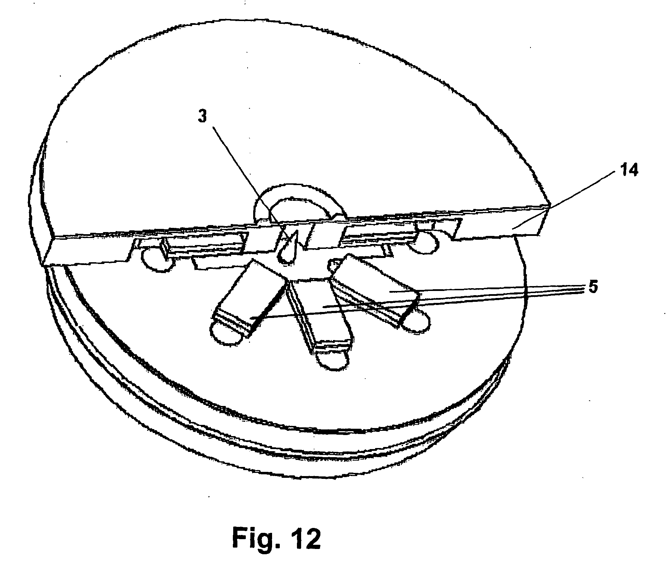

In der Fig. 12 ist eine Draufsicht auf eine Sensoreinheit 2 mit verschiedenen Sensorsystemen 5 dargestellt. Auf diese Weise sind Nachweissysteme für unterschiedliche Analyten gegeben, wobei entweder die Analyten gleichzeitig oder in einer zeitlich vorgebbaren Reihenfolge nachgewiesen werden.

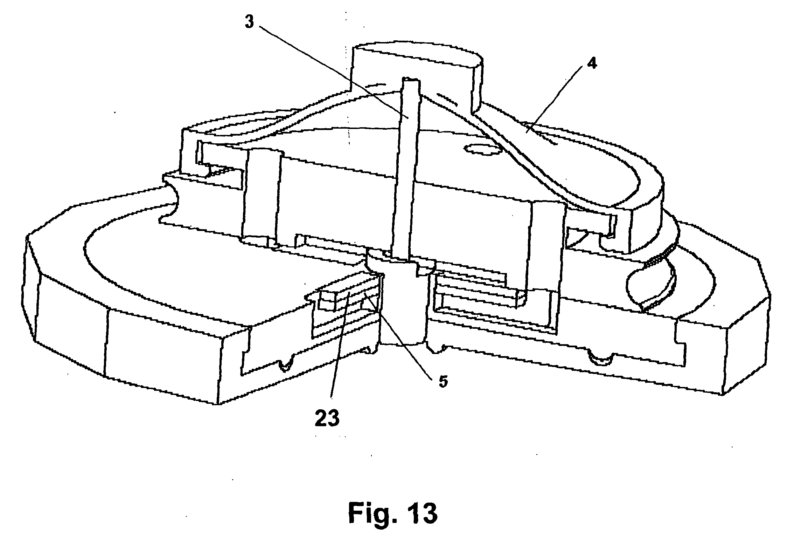

In der Fig. 13 ist eine schematische Darstellung einer Sensoreinheit 2 für den mehrmaligen Gebrauch im Schnitt angegeben. Durch einen drehbar einrastenden Zuführungskanal für die Körperflüssigkeit, insbesondere das Blut, wird der Fluss jeweils nur auf ein Sensorsystem 5 geleitet. Nach erfolgter Messung des Analyten dreht sich der Zuführungskanal um eine Rasterstellung weiter und öffnet den Blutkanal zum nächsten Sensorsystem 5. Auf diese Weise ist eine Sensoreinheit 2 auch für mehrmalige Bestimmungen des gleichen Analyten einsetzbar.

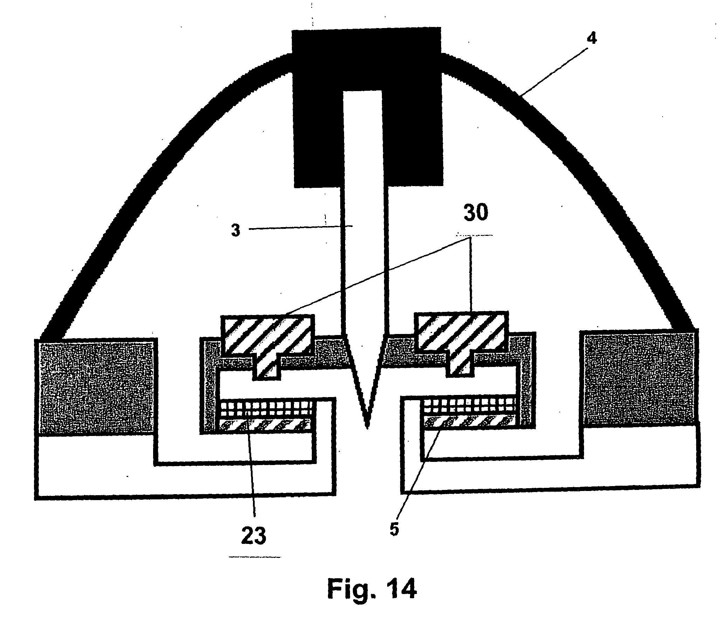

In der Fig. 14 ist eine schematische Darstellung einer Sensoreinheit 2 im Längsschnitt zu sehen, in der die nach dem Ansaugen der Körperflüssigkeit erfolgende quantitative Bestimmung von Analyten in der Körperflüssigkeit erst nach einer entsprechenden Aufarbeitung der Probe erfolgt. In der Filteranordnung 23 können Substanzen zur Probenvorbereitung enthalten sein. Zusätzlich können Vorratsbehälter 30 vorgesehen sein, die Substanzen zur Vorbereitung der Probe der Körperflüssigkeit und/oder zum Spülen enthalten, wobei diese Substanzen, beispielsweise durch Einwirkung von Druck über Ventile und Membranen, freigesetzt werden. In beiden Fällen kann durch eine Widerstandsmessung das korrekte Benetzen der Sensormatrix sichergestellt werden.

Die mit der erfindungsgemäßen Vorrichtung und ihren vorteilhaften Ausführungsformen sowie mit dem Verfahren hierzu durchführbare Probennahme und Analyse von Körperflüssigkeiten hat gegenüber den bisher bekannten Methoden folgende Vorteile:

Die benötigten Mengen an Körperflüssigkeit sind gering, ein weitgehend schmerzfreies Stechen wird durch Einsatz von Mikronadeln gewährleistet. Die Herstellungskosten sind durch Fertigung der Sensoreinheit im Spritzgussverfahren gering. Die Benutzung ist komfortabel durch die Integration von Stecheinheit und Sensorsystem in einer Einheit zum einmaligen Gebrauch sowie die Integration der Messeinheit in die Vorrichtung. Das Prinzip der Integration stellt darüber hinaus maximale Hygiene sicher. Die Vorrichtung hat eine ergonomische Gestalt und ein geringes Gewicht durch minimierten Energieverbrauch.

## Patentansprüche

1. Vorrichtung zur Probenahme und Analyse von Körperflüssigkeiten, umfassend
1.1 ein Handteil (1), welches eine pneumatische Antriebseinrichtung aufnimmt, und
1.2 eine Sensoreinheit (2), welche eine Mikronadel (3), die an einer elastischen Membran (4) befestigt ist, und mehrere Sensorsysteme (5) enthält, umfasst,
wobei das Handteil (1) und die Sensoreinheit (2) lösbar miteinander verbunden sind und die pneumatische Antriebseinrichtung einen Druck auf die elastische Membran (4) zum Einstechen der Mikronadel (3) in die Haut ausübt und anschließend durch Nachlassen des Druckes die elastische Membran (4) in die ursprüngliche Form zurückweicht, wodurch im Inneren der Sensoreinheit (2) ein Unterdruck erzeugt und Körperflüssigkeit zu einem Sensorsystem (5) geleitet wird und dort eine physikalische oder chemische Eigenschaftsänderung des Sensorsystems (5) bewirkt und wobei die Sensoreinheit (2) einen drehbar einrastenden Zuführungskanal für die Körperflüssigkeit aufweist, so dass die Körperflüssigkeit jeweils nur auf ein Sensorsystem (5) geleitet wird und nach erfolgter Messung eines Analyten der Zuführungskanal um eine Rasterstellung weiter drehbar ist, um den Zugang von Körperflüssigkeit zum nächsten Sensorsystem (5) zu öffnen.

2. Vorrichtung nach Anspruch 1, **dadurch gekennzeichnet, dass** das Handteil (1) eine Messeinheit (10) zur Bestimmung der in den Sensorsystemen (5) hervorgerufenen physikalischen oder chemischen Eigenschaftsänderungen enthält.

3. Vorrichtung nach Anspruch 2, **dadurch gekennzeichnet, dass** die Messeinheit (10) aus den physikalischen oder chemischen Eigenschaftsänderungen mindestens einen Konzentrationswert bestimmt.

4. Vorrichtung nach Anspruch 3, **dadurch gekennzeichnet, dass** ein an der Vorrichtung angebrachtes Display (12) den von der Messeinheit (10) bestimmten Konzentrationswert anzeigt.

5. Vorrichtung nach einem der Ansprüche 2 bis 4, **dadurch gekennzeichnet, dass** das Sensorsystem (5) und die Messeinheit (10) für ein optisches Messverfahren ausgelegt sind.

6. Vorrichtung nach Anspruch 5, **dadurch gekennzeichnet, dass** das Sensorsystem (5) und die Messeinheit (10) für ein fluoreszenzspektroskopisches Messverfahren ausgelegt sind.

7. Vorrichtung nach Anspruch 6, **dadurch gekennzeichnet, dass** das Sensorsystem (5) und die Messeinheit (10) für ein fluoreszenzspektroskopisches Messverfahren auf der Basis von Fluorescence Resonance Energy Transfer ausgelegt sind.

8. Vorrichtung nach einem der vorangehenden Ansprüche, **dadurch gekennzeichnet, dass** eine lösbare Verbindung (13) zwischen dem Handteil (1) und der Sensoreinheit (2) als Steck-, Klemm-, Schnapp-, Schraub- oder Klebeverbindung ausgebildet ist.

9. Vorrichtung nach einem der vorhergehenden Ansprüche, **dadurch gekennzeichnet, dass** die Sensoreinheit (2) eine Kontrolleinrichtung zum Nachweis ihrer korrekten, insbesondere ihrer ausreichenden Befüllung mit Körperflüssigkeit enthält.

10. Vorrichtung nach Anspruch 9, **dadurch gekennzeichnet, dass** die Kontrolleinrichtung eine elektrische Widerstandsmesseinheit umfasst.

11. Vorrichtung nach Anspruch 9 oder 10, **dadurch gekennzeichnet, dass** die Kontrolleinrichtung als Sollwert für eine ausreichende Befüllung ein Füllvolumen von weniger als 0.5 Mikroliter vorgibt.

12. Vorrichtung nach einem der vorhergehenden Ansprüche, **dadurch gekennzeichnet, dass** die für Bauteile der Vorrichtung benötigte elektrische Energie durch die Kopplung von piezoelektrischen oder elektromagnetischen Elementen mit der pneumatischen Antriebseinrichtung erzeugt wird.

13. Vorrichtung nach einem der vorhergehenden Ansprüche, **dadurch gekennzeichnet, dass** die Sensoreinheit (2) an der Unterseite einen Abdichtring (21) aufweist.

14. Vorrichtung nach Anspruch 13, **dadurch gekennzeichnet, dass** der Abdichtingring (21) an seiner Unterseite eine Klebeschicht aufweist.

15. Vorrichtung nach einem der vorhergehenden Ansprüche, **dadurch gekennzeichnet, dass** die Sensoreinheit (2) an der Unterseite durch eine abnehmbare oder eine vorgespannte, aufreißbare Abdeckfolie steril abgeschlossen ist.

16. Vorrichtung nach einem der vorhergehenden Ansprüche, **dadurch gekennzeichnet, dass** das Handteil (1) ein erstes Einstellglied (15) für eine variable Vorgabe der Einstichtiefe der Mikronadel (3) aufweist.

17. Vorrichtung nach einem der vorhergehenden Ansprüche, **dadurch gekennzeichnet, dass** das Handteil (1) ein zweites Einstellglied (17) für eine variable Vorgabe der Einstechgeschwindigkeit der Mikronadel (3) aufweist.

18. Vorrichtung nach einem der vorhergehenden Ansprüche, **dadurch gekennzeichnet, dass** das Handteil (1) ein drittes Einstellglied für eine variable Vorgabe der Ansauggeschwindigkeit von Körperflüssigkeit in die Sensoreinheit (2) aufweist.

19. Vorrichtung nach einem der vorhergehenden Ansprüche, **dadurch gekennzeichnet, dass** dem Sensorsystem (5) eine Filteranordnung (23) vorgeschaltet ist.

20. Vorrichtung nach einem der vorhergehenden Ansprüche, **dadurch gekennzeichnet, dass** dem mindestens einen Sensorsystem (5) ein oder mehrere Vorratsbehälter (30) mit Reagenzien und/oder Spüllösungen vorgeschaltet sind.

## Claims

1. Device for the sample-taking and analysis of bodily fluids comprising
1.1 a handset (1) which holds a pneumatic actuator device, and
1.2 a sensor unit (2) comprising a micro-needle (3) which is a attached to an elastic membrane (4), and several sensor systems (5)
whereby the handset (1) and the sensor unit (2) are connected to each other in a detachable manner and the pneumatic actuator device exerts a pressure on the elastic membrane (4) in order to inject the micro-needle (3) into the skin and then, through releasing the pressure (4) the elastic membrane (4) returns its original form, whereby inside the sensor unit (2) a vacuum is produced and bodily fluid is directed to a sensor system (5) where it brings about a physical or chemical change in the properties of the sensor system (5), and whereby the sensor unit (2) has a rotatably locking inlet channel for the bodily fluid so that the bodily fluid is only directed to one sensor system (5) in each case and after completed measurement of an analyte the inlet channel can be turned to another locking position in order to open access for the bodily fluid to the next sensor system (5).

2. Device in accordance with claim 1, **characterised in that** the handset (1) contains a measuring unit (10) for determining the physical or chemical property changes brought about in the sensor systems (5).

3. Device in accordance with claim 2, **characterised in that** the measuring unit (10) determines at least one concentration value from the physical and chemical property changes.

4. Device in accordance with claim 3, **characterised in that** a display (12) arranged on the device shows the concentration value determined by the measuring unit (10).

5. Device in accordance with any one of claims 2 to 4 **characterised in that** the sensor system (5) and the measuring unit (10) are designed for an optical measuring method.

6. Device in accordance with claim 5 **characterised in that** the sensor system (5) and the measuring unit (10) are designed for a fluorescence spectroscopic measuring method.

7. Device in accordance with claim 6, **characterised in that** the sensor system (5) and the measuring unit (10) are designed for a fluorescence spectroscopic measuring method based on Fluorescence Resonance Energy Transfer.

8. Device in accordance with any one of the preceding claims **characterised in that** a detachable connection (13) between the handset (1) and the sensor (2) is designed as a plug-type, clamp, snap, screw or adhesive connection.

9. Device in accordance with any one of the preceding claims **characterised in that** the sensor unit (2) contains a control device for detecting correct, more particularly complete, filling with bodily fluid.

10. Device in accordance with claim 9, **characterised in that** the control device comprises an electrical resistance measuring unit.

11. Device in accordance with claim 9 or 10, **characterised in that** the control device gives a filling volume less than 0.5 microlitres as the specified value for sufficient filling.

12. Device in accordance with any one of the preceding claims **characterised in that** electrical energy required for the components of the device is produced by the coupling of piezoelectrical or electromagnetic elements with the pneumatic actuator unit.

13. Device in accordance with any one of the preceding claims **characterised in that** the sensor unit (2) has a sealing ring (21) on its lower side.

14. Device in accordance with claim 13 **characterised in that** the sealing ring (21) has an adhesive layer on its underside.

15. Device in accordance with any one of the preceding claims **characterised in that** the sensor unit (2) is sealed on its underside in a sterile manner by means of a removable or pre-tensioned tear-off cover film.

16. Device in accordance with any one of the preceding claims **characterised in that** the handsets (1) has a first adjusting element (15) for variable determination of the injection depth of the micro-needle (3).

17. Device in accordance with any one of the preceding claims, **characterised in that** the handset (1) has a second adjusting element (17) for variable determination of the injection speed of the micro-needle (3).

18. Device in accordance with any one of the preceding claims **characterised in that** the handset (1) has a third adjusting element for variable determination of the rate of suction of the bodily fluid into the sensor unit (2).

19. Device in accordance with any one of the preceding claims, **characterised in that** a filter arrangement (23) precedes the sensor system (5).

20. Device in accordance with any one of the preceding claims **characterised in that** one or more storage containers (30) with reagents and/or flushing solutions precede(s) the at least one sensor system (5).

## Revendications

1. Dispositif de prélèvement d'échantillons et d'analyse de liquides physiologiques, comportant
1.1 une pièce manuelle (1) recevant un dispositif de propulsion pneumatique et
1.2 une unité de détection (2) englobant une micro-aiguille (3) qui est fixée à une membrane élastique (4) et contient plusieurs systèmes de détection (5),
la pièce manuelle (1) et l'unité de détection (2) étant reliées entre elles de manière dissociable et le dispositif de propulsion pneumatique exerçant une pression sur la membrane élastique (4) pour piquer la micro-aiguille (3) dans la peau et la membrane élastique (4) reprenant ensuite par relâchement de la pression sa forme d'origine, ce qui génère à l'intérieur de l'unité de détection (2) une dépression et conduit le liquide physiologique à un système de détection (5) et provoque là une modification des propriétés physiques ou chimiques du système de détection (5), l'unité de détection (2) comportant un canal d'acheminement s'enclenchant en rotation pour le liquide physiologique, de sorte que le liquide physiologique n'est conduit respectivement qu'à un système de détection (5) et qu'une fois la mesure d'un analyte réalisée, le canal d'acheminement peut être tourné à raison d'une position d'enclenchement plus loin afin d'ouvrir l'accès de liquide physiologique vers le système de détection suivant (5).

2. Dispositif selon la revendication 1, **caractérisé en ce que** la pièce manuelle (1) comprend une unité de mesure (10) pour déterminer les modifications de propriétés physiques ou chimiques provoquées dans les systèmes de détection (5).

3. Dispositif selon la revendication 2, **caractérisé en ce que** l'unité de mesure (10) définit à partir des modifications de propriétés physiques ou chimiques au moins une valeur de concentration.

4. Dispositif selon la revendication 3, **caractérisé en ce qu'**un afficheur (12) installé sur le dispositif affiche la valeur de concentration définie par l'unité de mesure (10).

5. Dispositif selon une des revendications 2 à 4, **caractérisé en ce que** le système de détection (5) et l'unité de mesure (10) sont conçus pour un procédé de mesure optique.

6. Dispositif selon la revendication 5, **caractérisé en ce que** le système de détection (5) et l'unité de mesure (10) sont conçus pour un procédé de mesure spectroscopique à fluorescence.

7. Dispositif selon la revendication 6, **caractérisé en ce que** le système de détection (5) et l'unité de mesure (10) sont conçues pour un procédé de mesure spectroscopique à fluorescence basé sur le transfert d'énergie de résonance de fluorescence.

8. Dispositif selon une des revendications précédentes, **caractérisé en ce qu'**une liaison dissociable (13) est établie entre la pièce manuelle (1) et l'unité de détection (2) sous forme d'une liaison par fichage, serrage, clippage, vissage ou collage.

9. Dispositif selon une des revendications précédentes, **caractérisé en ce que** l'unité de détection (2) comporte un dispositif de contrôle pour mettre en évidence son remplissage correct, notamment suffisant, par du liquide physiologique.

10. Dispositif selon la revendication 9, **caractérisé en ce que** le dispositif de contrôle comprend une unité de mesure de résistance électrique.

11. Dispositif selon la revendication 9 ou 10, **caractérisé en ce que** le dispositif de contrôle prescrit comme valeur théorique pour un remplissage suffisant un volume de remplissage de moins de 0,5 microlitre.

12. Dispositif selon une des revendications précédentes, **caractérisé en ce que** l'énergie électrique nécessaire pour les composantes du dispositif est générée par le couplage d'éléments piézoélectriques ou électromagnétiques au dispositif de propulsion pneumatique.

13. Dispositif selon une des revendications précédentes, **caractérisé en ce que** l'unité de détection (2) présente sur sa face inférieure une bague d'étanchéité (21).

14. Dispositif selon la revendication 13, **caractérisé en ce que** la bague d'étanchéité (21) présente sur sa face inférieure une couche collante.

15. Dispositif selon une des revendications précédentes, **caractérisé en ce que** l'unité de détection (2) est fermée de manière stérile au niveau de sa face inférieure par un film de recouvrement retirable ou précontraint et arrachable.

16. Dispositif selon une des revendications précédentes, **caractérisé en ce que** la pièce manuelle (1) présente un premier élément de mise au point (15) pour la prescription variable de la profondeur de piquage de la micro-aiguille (3).

17. Dispositif selon une des revendications précédentes, **caractérisé en ce que** la pièce manuelle (1) présente un deuxième élément de mise au point (17) pour la prescription variable de la vitesse de piquage de la micro-aiguille (3).

18. Dispositif selon une des revendications précédentes, **caractérisé en ce que** la pièce manuelle (1) présente un troisième élément de mise au point pour la prescription variable de la vitesse d'aspiration de liquide physiologique dans l'unité de détection (2).

19. Dispositif selon une des revendications précédentes, **caractérisé en ce qu'**en amont du système de détection (5) est branché un dispositif de filtrage (23).

20. Dispositif selon une des revendications précédentes, **caractérisé en ce qu'**en amont de l'au moins un système de détection (5) sont branchés un ou plusieurs récipients de réserve (30) contenant des réactifs et/ou des solutions de rinçage.
